(19) Europäisches Patentamt
European Patent Office
Office européen des brevets

(11) Veröffentlichungsnummer : **0 181 283**
**B1**

(12) # EUROPÄISCHE PATENTSCHRIFT

(45) Veröffentlichungstag der Patentschrift : **28.09.88**

(51) Int. Cl.[4] : **C 07 C 65/105**, C 07 C 65/24, B 41 M 5/12

(21) Anmeldenummer : **85810471.4**

(22) Anmeldetag : **16.10.85**

(54) **Isomerengemische von Metallsalicylaten, ihre Herstellung und Verwendung.**

(30) Priorität : **22.10.84 CH 5046/84**

(43) Veröffentlichungstag der Anmeldung : **14.05.86 Patentblatt 86/20**

(45) Bekanntmachung des Hinweises auf die Patenterteilung : **28.09.88 Patentblatt 88/39**

(84) Benannte Vertragsstaaten : **BE CH DE FR GB IT LI SE**

(56) Entgegenhaltungen : **CH-A- 479 526**

(73) Patentinhaber : **CIBA-GEIGY AG**
**Klybeckstrasse 141**
**CH-4002 Basel (CH)**

(72) Erfinder : **Nachbur, Hermann**
**Mischelistrasse 21**
**CH-4153 Reinach (CH)**

**EP 0 181 283 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Isomerengemische von Metallsalicylaten, Verfahren zu ihrer Herstellung und ihre Verwendung in druckempfindlichen oder wärmeempfindlichen Aufzeichnungsmaterialien. Die Metallsalicylate entsprechen der Formel

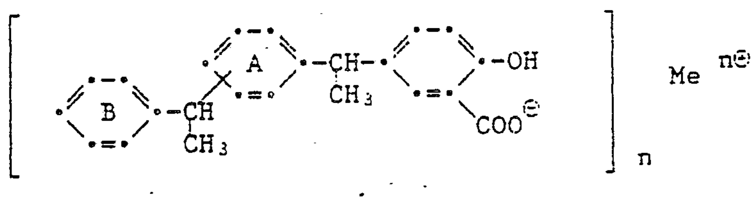

in der

Me ein n-wertiges Metallion und

n 2, 3 oder 4 bedeuten und die Ringe A und B unabhängig voneinander unsubstituiert oder durch Halogen, Niederalkyl, Niederalkoxy oder einen α-Methylbenzylrest substituiert sind.

Die bei den definitionsgemässen Metallsalzen zweifach bis vierfach vorkommenden Salicylatbestandteile können gleich oder verschieden sein. Vorzugsweise sind sie jeweils identisch.

Niederalkyl und Niederalkoxy stellen in der Regel Gruppen dar, die 1 bis 5, insbesondere 1 bis 3 Kohlenstoffatome aufweisen, wie z. B. Methyl, Ethyl, Isopropyl, sek.-Butyl, tert.-Butyl, Amyl oder Isoamyl bzw. Methoxy, Ethoxy, Isopropoxy, n-Butoxy oder tert.-Butoxy.

Halogen bedeutet beispielsweise Fluor, Jod, Brom oder vorzugsweise Chlor.

Die erfindungsgemässen Metallsalze leiten sich vorteilhafterweise von 2-, 3- oder 4-wertigen Metallen mit einem Atomgewicht von 24 bis 210, vorzugsweise 26 bis 120 ab. Beispiele derartiger Metalle sind Aluminium, Barium, Blei, Cadmium, Calcium, Chrom, Eisen, Gallium, Kobalt, Kupfer, Magnesium, Mangan, Molybdän, Nickel, Quecksilber, Silber, Strontium, Tantal, Titan, Vanadium, Wolfram, Zink, Zinn und Zirkonium. Dabei sind Aluminium, Titan, Vanadium, Zinn und insbesondere Zink bevorzugt.

Die

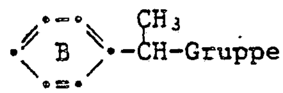

befindet sich vorteilhafterweise in p-Stellung zur Ethylidengruppe.

Die Ringe A und B sind vorzugsweise nicht weiter substituiert. Falls die Ringe A und B Substituenten aufweisen, sind sie in erster Linie durch Halogen, Methyl, Methoxy oder α-Methylbenzyl weiter substituiert. Pro Benzolring A oder B können vorteilhafterweise 1 oder 2 Substituenten zusätzlich vorhanden sein. Der α-Methylbenzylrest ist in der Regel im Benzolring B vorhanden.

Praktisch wichtige Farbentwickler, die vorteilhaft erfindungsgemäss eingesetzt und nach den nachstehenden Verfahren hergestellt werden können, sind Isomerengemische von Aluminium- oder vorzugsweise Zinksalzen einer substituierten Salicylsäureverbindung der Formel

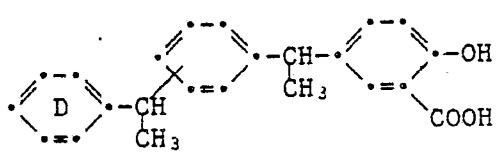

in der der Ring D unsubstituiert oder durch α-Methylbenzyl substituiert ist.

Unter den Salicylsäureverbindungen der Formel (2) sind diejengien, in denen der Ring D unsubstituiert ist, bevorzugt.

Im Vordergrund des Interesses steht als Farbentwickler das Zinksalz der 5-[α-Methyl-4'(α-methylbenzyl)-benzyl]-salicylsäure.

Die Metallverbindungen der Formel (1) stellen eine neue Klasse von Metallsalzen dar, welche als Farbentwickler oder Elektronenakzeptoren für elektronenabgebende Farbbildner geeignet sind.

Die Metallsalicylate der Formel (1) werden dadurch hergestellt, dass man 1 Mol des Salzes eines n-wertigen Metalls einer anorganischen Säure oder einer niederen aliphatischen Carbonsäure mit n Mol einer Salicylsäureverbindung der Formel

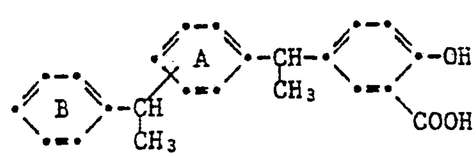

worin n, A und B die angegebene Bedeutung haben, umsetzt.

Die Umsetzung erfolgt vorteilhafterweise in einer alkalischen Lösung der verwendeten Salicylsäureverbindung und vorzugsweise in Gegenwart einer Alkaliverbindung, wie z. B. Alkalimetallhydroxide, -carbonate oder -bicarbonate oder Ammoniumhydroxid, Ammoniumcarbonat oder Ammoniumhydrogencarbonat.

Die Metallisierung kann schon bei einer Temperatur von 10 bis 25 °C vorgenommen werden. In gewissen Fällen und besonders bei Verwendung von organischen Aluminiumsalzen ist es nötig bei höheren Temperaturen, vorzugsweise 70 bis 200 °C zu arbeiten.

Man kann die Reaktionspartner aber auch in einer Schmelze umsetzen. Als Schmelzmittel eignen sich z. B. Salze niederer Fettsäuren, wie z. B. Natriumacetat, Amide niederer Fettsäuren, wie z. B. Acetamid, ferner Harnstoff oder Thioharnstoff oder deren N-Substitutionsprodukte.

Als Metallabgebende Mittel verwendet man zweckmässig die Metallsalze von Mineralsäuren oder Carbonsäuren mit 1 bis 6 Kohlenstoffatomen, insbesondere Sulfate, Halogenide (Chloride), Nitrate, Formiate, Acetate, Propionate, Oxalate oder Citrate.

Als Beispiele für anorganische Metallsalze seien die Zinksalze Zinkchlorid, Zinksulfat oder Zinknitrat sowie auch Aluminiumsulfat genannt.

Organische Metallsalze sind z. B. Zinkdiacetat, Zinkoxalat, Aluminiumtriisopropylat oder Aluminiumsek. butylat.

Anstelle der genannten Zinksalze können auch Zinkoxid oder Zinkcarbonat verwendet werden, wobei in diesem Falle die Umsetzung mit der Salicylsäure, vorzugsweise in Gegenwart von Ammoniumformiat durchgeführt wird.

Die substituierten Salicylsäureverbindungen der Formel (3) und deren Herstellung sind neu.

Die Herstellung kann dadurch erfolgen, dass man 1 Mol Salicylsäure mit mindestens 1 Mol eines 1-Phenylethanols der Formel

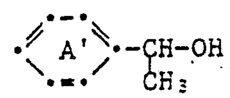

(4)

und mindestens 1 Mol eines 1-Phenylethanols der Formel

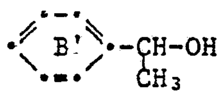

(5)

umsetzt, worin die Ringe A' und B' unsubstituiert oder durch Halogen, Niederalkyl oder Niederalkoxy substituiert sind.

Vorzugsweise sind die Ethanolkomponenten der Formeln (4) und (5) identisch.

Als Beispiele für geeignete 1-Phenylethanol-komponenten der Formeln (4) und (5) seien genannt : 1-Phenylethanol, 1-Tolylethanol, 1-Xylylethanol oder 1-(Chlorphenyl)-ethanol.

Zur Herstellung der Salicylsäureverbindungen der Formel (3) arbeitet man zweckmässigerweise in einem nicht an der Kondensation teilnehmenden, organischen Lösungsmittel und in Gegenwart eines sauren Katalysators, bei einer Temperatur zwischen 20 °C und der Rückflusstemperatur des Reaktionsmediums, vorzugsweise bei 80 ° bis 150 °C.

Die Reaktionsdauer hängt von der Temperatur ab und liegt in der Regel zwischen 1/2 und 5 Stunden, vorzugsweise 1 bis 3 Stunden.

Geeignete organische Lösungsmittel, die das Reaktionsmedium bilden, sind cycloaliphatische oder vorzugsweise aromatische Kohlenwasserstoffe, wie z. B. Cyclohexan, Benzol, Toluol oder Xylol ; Chlorkohlenwasserstoffe, wie z. B. Ethylenchlorid, Tetrachlorethylen oder Chlorbenzole, wie z. B. Chlorbenzol, Chlortoluol oder Dichlorbenzol ; cyclische Ether, wie z. B. Dioxan oder Tetrahydrofuran ; Dimethylsulfoxid oder Nitrile aliphatischer Monocarbonsäuren, wie z. B. Acetonitril, Propionitril oder Butyronitril. Auch Mischungen der genannten Lösungsmittel können verwendet werden. Bevorzugte Lösungsmittel sind Chlorbenzol, Chlortoluol und besonders Toluol.

Als saure Katalysatoren eignen sich aromatische Sulfonsäuren, wie z. B. Benzolsulfonsäure, Chlorbenzolsulfonsäure, Toluolsulfonsäure, Chlortoluolsulfonsäure oder Xylolsulfonsäure.

Bevorzugt sind in situ hergestellte aromatische Sulfonsäuren und insbesondere Toluolsulfonsäure. Die Herstellung der Toluolsulfonsäure in situ erfolgt z. B. wie in Chemical Abstract, Vol. 79 (1969), 106228r beschrieben. p-Toluolsulfonsäure ist ebenfalls bevorzugt. In letzterem Falle kann auf die Verwendung eines Lösungsmittels verzichtet werden.

Nach der Kondensation der Salicylsäure mit den anderen Ausgangsstoffen der Formeln (4) und (5) kann die substituierte Salicylsäureverbindung der Formel (3) direkt zur Herstellung des Metallsalzes der Formel (1) weiterverwendet werden. Falls eine Isolierung der substituierten Salicylsäureverbindung der Formel (3) erwünscht ist, wird z. B. die saure Lösung des Umsetzungsproduktes zuerst mit einer wässerigen Natriumhydroxydlösung neutralisiert und dann die neutrale Lösung mit einer niederen

Carbonsäure oder einer anorganischen Säure angesäuert, worauf das Produkt in Form eines Oels ausfällt und abgetrennt wird.

Eine besonders zweckmässige Ausführungsform zur Herstellung der Metallverbindungen der Formel (1), in der Me das Zinkion ist, besteht darin, dass man in einem Reaktionsmedium bestehend aus Toluol und in situ hergestellter Toluolsulfonsäure etwa 1 Mol, vorzugsweise 1,0 bis 1,2 Mol Salicylsäure mit mehr als 2 Mol 1-Phenylethanol zum Sieden erhitzt und unter Rückflusstemperatur vorzugsweise während 1/2 bis 2 1/2 Stunden kondensiert. Darauf wird das Toluol entfernt, die erhaltene Salicylsäureverbindung der Formel (3) in einer wässerigen Natriumhydroxidlösung gelöst und die alkalische Lösung mit einem anorganischen Zinksalz, vorzugsweise Zinkchlorid behandelt, worauf das erhaltene Zinksalz der Salicylsäureverbindung der Formel (3) isoliert wird.

Durch Lösen des Zinksalzes in einem polaren organischen Lösungsmittel, z. B. Aceton und Ansäuern der Lösung mit einer verdünnten anorganischen Säure, z. B. Salzsäure, kann die metallfreie Salicylsäureverbindung leicht zurückgebildet werden.

Ein grosser Vorteil der vorliegenden Erfindung liegt darin, dass durch die Wahl der Ausgangsprodukte, insbesondere Salicylsäure und 1-Phenylethanol, überraschenderweise anstatt eines Veresterungsproduktes der Salicylsäure eine neue, leicht zugängliche ringsubstituierte Salicylsäureverbindung bereitgestellt werden konnte, deren Metallsalz und insbesondere das Zinksalz einen ausgezeichneten und wirtschaftlich günstigen Farbentwickler für Farbbildner darstellt, der sowohl in druckempfindlichen als auch in wärmeempfindlichen Aufzeichungsmaterialien eingesetzt werden kann.

Die Metallsalicylate der Formel (1) sind praktisch farb- und geruchlos und mit den üblichen Farbbildnern sehr reaktiv, so dass damit spontane, beständige (lagerstabile) und nicht verblassende Aufzeichnungen oder Kopien erhalten werden.

Die im erfindungsgemässen Aufzeichnungsmaterial oder Kopiermaterial in Betracht kommenden Farbbildner sind bekannte farblose oder schwach gefärbte chromogene Stoffe, die, sofern sie mit den Metallverbindungen der Formel (1) in Kontakt kommen, farbig werden oder die Farbe ändern. Es können Farbbildner oder deren Mischungen verwendet werden, welche z. B. den Klassen der Azomethine, Fluorane, Benzofluorane, Phthalide, Azaphthalide, Spiropyrane, Spirodipyrane, Leukoauramine, Chinazoline, Triarylmethanleukofarbstoffe, Carbazolylmethane, Chromenoindole, Rhodaminlaktame, Chromenopyrazole, Phenoxazine, Phenothiazine sowie der Chromeno- oder Chromanofarbbildner angehören. Als Beispiele solcher geeigneter Farbbildner seien genannt : Kristallviolettlacton, 3,3-(Bisaminophenyl)-phthalide, 3,3-(Bis-substituierte-indolyl)-phthalide, 3-(Aminophenyl)-3-indolylphthalide, 3-(Aminophenyl)-3-indolylazaphthalide, 6-Dialkylamino-2-n-octylamino-fluorane, 6-Dialkylamino-2-arylamino-fluorane, z. B. 6-Diethylamino-2-(2'-chlorphenylamino)-fluoran, 6-Dibutylamino-2-(2'-chlorphenylamino)-fluoran ; 6-Dialkylamino-3-methyl-2-aryl-amino-fluorane, z. B. 2-Anilino-3-methyl-6-diethylaminofluoran oder 2-(2',4'-Dimethylanilino)-3-methyl-6-diethylaminofluoran, 6-Dialkylamino-2- oder 3-niederalkyl-fluorane, 6-Dialkylamino-2-dibenzylamino-fluorane, 6-Pyrrolidino-2-dibenzylaminofluoran, 6-N-Cyclohexyl-N-niederalkyl-3-methyl-2-arylamino-fluorane, 6-Pyrrolidino-2-arylamino-fluorane, Bis-(aminophenyl)-furyl- oder -phenyl- oder -carbazolyl-methane, 3'-Phenyl-7-dialkylamino-2,2'-spirodibenzopyrane, Bisdialkylaminobenzhydrol-alkyl- oder -aryl-sulfinate, Benzoyldialkylamino-phenothiazine oder -phenoxazine.

Die Metallsalicylate der Formel (1) eignen sich als Farbentwickler für druckempfindliches oder für wärmeempfindliches Aufzeichnungsmaterial, das sowohl Kopier- als auch Registriermaterial sein kann.

Ein druckempfindliches Material besteht beispielsweise aus mindestens einem Paar von Blättern, die mindestens einen Farbbildner, gelöst in einem organischen Lösungsmittel, und ein Metallsalicylat der Formel (1) als Entwickler enthalten.

Die Entwickler werden vorzugsweise in Form einer Schicht auf die Vorderseite des Empfangsblattes aufgebracht.

Die Metallsalicylate der Formel (1) können für sich allein, als Mischungen oder in Mischungen mit bekannten Entwicklern eingesetzt werden. Typische Beispiele für bekannte Entwickler sind Aktivton-Substanzen, wie Attapulgus-Ton, Säureton, Bentonit, Montmorillonit ; aktivierter Ton, z. B. säureaktiviertes Bentonit oder Montmorillonit ; ferner Halloysit, Zeolith, Siliciumdioxid, Aluminiumoxid, Aluminiumsulfat, Aluminiumphosphat, Zinkchlorid, Zinknitrat, Kaolin oder irgendein beliebiger Ton oder sauer reagierende, organische Verbindungen, wie z. B. gegebenenfalls ringsubstituierte Phenole, 3,5-Bis-($\alpha$,$\alpha$-dimethyl-benzyl) salicylsäure, 3,5-Bis-($\alpha$-methyl-benzyl) salicylsäure oder Salicylsäureester und deren Metallsalze ; ferner ein sauer reagierendes, polymeres Material, wie z. B. ein phenolisches Polymerisat, ein Alkylphenolacetylenharz, ein Maleinsäure-Kolophonium-Harz oder ein teilweise oder vollständig hydrolysiertes Polymerisat aus Maleinsäureanhydrid und Styrol, Ethylen oder Vinymethylether, oder Carboxypolymethylen.

Die Entwickler können zusätzlich auch mit an sich unreaktiven oder wenig reaktiven Pigmenten oder weiteren Hilfsstoffen wie Kieselgel oder UV-Adsorbern, wie z. B. 2-(2-Hydroxyphenyl)- benzotriazolen gemischt eingesetzt werden. Beispiele für solche Pigmente sind : Talk, Titandioxid, Zinkoxid, Kreide ; Tone wie Kaolin, sowie organische Pigmente, z. B. Harnstoff-Formaldehyd-Kondensate (BET-Oberfläche 2-75 $m^2/g$) oder Melamin-Formaldehyd-Kondensationsprodukte.

Der Farbbildner liefert an den Punkten, an denen er mit dem Entwickler in Kontakt kommt, eine gefärbte Markierung. Um zu verhindern, dass die Farbbildner, die in dem druckempfindlichen Aufzeichnungsmaterial enthalten sind, frühzeitig aktiv werden, werden sie in der Regel von dem Entwickler

getrennt. Dies kann zweckmässig erzielt werden, indem man die Farbbildner in schaum-, schwamm- oder bienenwabenartige Strukturen einarbeitet. Vorzugsweise sind die Farbbildner in Mikrokapseln eingeschlossen, die sich in der Regel durch Druck zerbrechen lassen.

Wenn die Kapseln durch Druck, beispielsweise mittels eines Bleistiftes zersprengt werden und die Farbbildnerlösung auf diese Weise auf ein benachbartes Blatt übertragen wird, das mit dem Metallsalicylat der Formel (1) beschichtet ist, wird eine farbige Stelle erzeugt. Diese Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z. B. polyhalogeniertes Paraffin oder Diphenyl, wie Chlorparaffin, Monochlordiphenyl oder Trichlordiphenyl, ferner Tricresylphosphat, Di-n-butylphthalat ; aromatische Ether, wie Benzylphenylether ; Kohlenwasserstofföle, wie Paraffin oder Kerosin, alkylierte Derivate, (z. B. mit Isopropyl oder Isobutyl) von Diphenyl, Diphenylalkane, Naphthalin oder Triphenyl, Dibenzyltoluol, Dodecylbenzol, Terphenyl, partiell hydriertes Terphenyl, benzylierte Xylole oder weitere chlorierte oder hydrierte, kondensierte, aromatische Kohlenwasserstoffe. Oft werden Mischungen verschiedener Lösungsmittel eingesetzt, um eine optimale Löslichkeit für die Farbbildung, eine rasche und intensive Färbung und eine für die Mikroverkapselung günstiges Viskosität zu erreichen.

Die Kapselwände können durch Koazervationskräfte gleichmässig um die Tröpfchen der Farbbildnerlösung herum gebildet werden, wobei das Einkapselungsmaterial, z. B. aus Gelatine und Gummiarabikum bestehen kann, wie dies z. B. in der US-Patentschrift 2 800 457 beschrieben ist. Die Kapseln können vorzugsweise auch aus einem Aminoplast oder aus modifizierten Aminoplasten durch Polykondensation gebildet werden, wie es in den britischen Patentschriften 989 264, 1 156 725, 1 301 052 und 1 355 127 beschrieben ist. Ebenfalls geeignet sind Mikrokapseln, welche durch Grenzflächenpolymerisation gebildet werden, wie z. B. Kapseln aus Polyester, Polycarbonat, Polysulfonamid, Polysulfonat, besonders aber aus Polyamid oder Polyurethan.

Die Farbbildner enthaltenden Mikrokapseln können kombiniert mit den Farbentwicklern zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten, d. h. der Entwickler und durch das Trägermaterial. Bevorzugt wird eine Anordnung, bei der der eingekapselte Farbbildner in Form einer Schicht auf der Rückseite eines Uebertragungsblattes und der erfindungsgemäss zu verwendende Entwickler der Formel (1) in Form einer Schicht auf der Vorderseite eines Empfangsblattes vorhanden sind.

Eine andere Anordnung der Bestandteile besteht darin, dass die Farbbildner enthaltenden Mikrokapseln und der Entwickler in oder auf dem gleichen Blatt in Form einer oder mehrerer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papierbeschichtungsmittel, wie Gummiarabikum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylmono- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Metallverbindungen der Formel (1) können auch als Entwickler in einem thermoreaktiven Aufzeichnungsmaterial verwendet werden. Dieses enthält in der Regel mindestens einen Schichtträger, einen Farbbildner, einen Entwickler und gegebenenfalls auch ein Bindemittel und/oder Wachse.

Thermoreaktive Aufzeichnungssysteme umfassen z. B. wärmeempfindliche Aufzeichnungs- und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und Messinstrumenten verwendet, wie z. B. Elektrocardiographen. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst oder dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Entwickler in Kontakt und es entwickelt sich sofort die erwünschte Farbe.

Die Entwickler der Formel (1) können auch in wärmeempfindlichen Aufzeichnungsmaterialien für sich allein, als Mischungen oder in Mischung mit bekannten Entwicklern eingesetzt werden.

Bekannt sind für diesen Zweck die gleichen Entwickler, wie sie in druckempfindlichen Papieren verwendet werden, sowie auch phenolische Verbindungen, wie z. B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäuremethyl-, -ethyl-, n-butyl- oder -benzylester, 4-Hydroxyacetophenon, 2,2'-Dihydroxydiphenyl, 4,4'-Isopropylidendiphenol, 4,4'-Isopropyliden-bis-(2-methylphenol), 4-Hydroxy-diphenylsulfon, 4'-Methyl-4-hydroxydi-

phenylsulfon, 2,4-Dihydroxydiphenylsulfon, 4,4'-Bis-(hydroxyphenyl)valeriansäure, 2,4-Dihydroxybenzophenon, Hydrochinon, Pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z. B. Weinsäure, Oxalsäure, Maleinsäure, Zitronensäure, Citraconsäure oder Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Farbbildner und der Entwickler in Wasser unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserlösliche oder mindestens in Wasser quellbare Bindemittel sind z. B. hydrophile Polymerisate, wie Polyvinylalkohol, Polyacrylsäure, Hydroxyethylcellulose, Methylcellulose, Carboxymethylcellulose, Polyacrylamid, Polyvinylpyrrolidon, carboxylierte Butadien-Styrolcopolymerisate, Gelatine, Stärke oder veretherte Maisstärke.

Wenn der Farbbildner und der Entwickler in zwei getrennten Schichten vorliegen, können in Wasser unlösliche Bindemittel, d. h. in nichtpolaren oder nur schwach polaren Lösungsmitteln lösliche Bindemittel, wie z. B. Naturkautschuk, synthetischer Kautschuk, chlorierter Kautschuk, Alkyldharze, Polystyrol, Styrol/Butadien-Mischpolymerisate, Polymethylacrylate, Ethylcellulose, Nitrocellulose und Polyvinylcarbazol verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z. B. Talk, Titandioxid, Zinkoxid, Aluminiumoxid, Aluminiumhydroxid, Calciumcarbonat (z. B. Kreide), Tone wie Kaolin, sowie organische Pigmente, wie z. B. Harnstoffformaldehyd-oder Melaminformaldehydpolymerisate enthalten. Um zu bewirken, dass nur innerhalb eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Dimethylterephthalat, Phthalsäureanhydrid, Metallchloride, Metallstearate z. B. Zinkstearat, Phthalsäurenitril oder andere entsprechende, schmelzbare Produkte, welche das gleichzeitige schmelzen des Farbbildners und des Entwicklers induzieren, zugesetzt werden. Bevorzugt enthalten thermographische Aufzeichnungsmaterialien Wachse, z. B. Carnaubawachs, Montanawachs, Paraffinwachs, Polyethylenwachs oder Kondensate höherer Fettsäureamide und Formaldehyd oder Kondensate höherer Fettsäuren und Ethylendiamin.

In den folgenden Herstellungs- und Anwendungsbeispielen beziehen sich die angegebenen Prozentsätze, wenn nichts anderes angegeben ist, auf das Gewicht und Teile sind Gewichtsteile.

## Herstellungsbeispiele

### Beispiel 1

250 g Toluol werden unter Rückfluss erhitzt. Hierzu lässt man im Verlaufe von 50 bis 60 Minuten 7,3 g 96 %iger Schwefelsäure zutropfen und entfernt das entstehende Wasser azeotrop. Alsdann kühlt man auf 60 °C ab und gibt 250 g 1-Phenylethanol und 155 g Salicylsäure hinzu. Man erwärmt das Gemisch auf Rückflusstemperatur und entfernt das bei der Kondensation gebildete Wasser laufend azeotrop. Danach wird das Toluol abdestilliert und der Rückstand mit 1 190 ml einer wässrigen 1n Natriumhydroxidlösung verrührt. Die erhaltene Lösung wird einige Stunden bei Raumtemperatur stehen gelassen, worauf das an der Oberfläche abgeschiedene Oel abgetrennt wird. Hierauf lässt man die Lösung bei 10-15 °C und unter starkem Rühren in eine Lösung von 200 g Zinkchlorid in 600 ml Wasser zutropfen, worauf der erhaltene weisse Niederschlag abfiltriert und in 1 kg Toluol gelöst wird. Die Toluollösung wird bei 60 °C unter reduziertem Druck eingedampft. Man erhält 233 g eines wasserfreien pulverförmigen Produktes, welches das Zinksalz der Formel

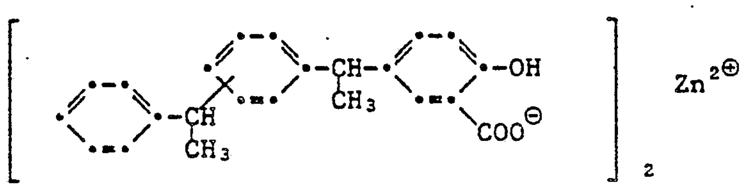 (11)

darstellt.

Dieses Produkt schmilzt bei 130-190 °C.

Elementaranalyse :

ber. : C = 73,06 % H = 5,6 % O = 12,7 % Zn = 8,64 %

gef. : C = 71,8 % H = 5,7 % O = 12,8 % Zn = 9,7 %

### Beispiel 2

Eine Lösung von 15,12 g des in Beispiel 1 hergestellten Zinksalzes der Formel (11) in 110 ml Aceton

wird mit 50 ml einer wässerigen 1n-Salzsäurelösung versetzt. Hierauf wird die saure Lösung während 1 Stunde bei Rückflusstemperatur gehalten und danach unter Vakuum eingedampft. Der Rückstand wird in 80 ml Toluol aufgenommen, wobei sich 2 Phasen bilden. Die Toluolphase wird abgetrennt und unter Vakuum eingedampft. Man erhält 13,1 g eines viskosen Produktes, welches die Salicylsäureverbindung der Formel

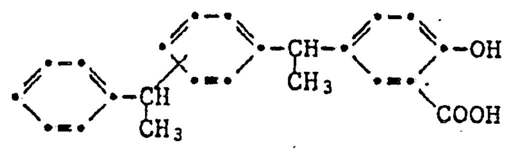

(12)

darstellt.

Elementaranalyse :
ber. : C = 79,74 % H = 6,4 %
gef. : C = 80,6 % H = 6,8 %

Beispiel 3

160 g o-Chlortoluol werden auf 110 °C erhitzt. Hierzu lässt man innert 35 Minuten 3,65 g 96 %ige Schwefelsäure zutropfen und entfernt das entstehende Wasser bei reduziertem Druck. Alsdann kühlt man auf 60 °C ab und gibt 125 g 1-Phenylethanol und 77,5 g Salicylsäure hinzu. Man erwärmt das Gemisch auf 83 °C und entfernt das bei der Kondensation gebildete Wasser laufend azeotrop, wobei man bei reduziertem Druck arbeitet. Danach wird das o-Chlortoluol abdestilliert und der Rückstand mit 560 ml einer wässrigen 1n Natriumhydroxydlösung verrührt und anschliessend mit 1 l Wasser verdünnt. Die erhaltene Lösung wird einige Stunden bei Raumtemperatur stehen gelassen, worauf das an der Oberfläche abgeschiedene Oel abgetrennt wird. Hierauf lässt man die Lösung bei 10-15 °C und unter Rühren in eine Lösung von 47,7 g Zinkchlorid in 300 ml Wasser zutropfen, worauf der erhaltene Niederschlag abfiltriert und abgepresst wird. Der feuchte Nutschkuchen wird nochmals in 1 l Wasser aufgeschlämmt und wieder abfiltriert, abgepresst und bei 100 °C in Vakuum getrocknet. Man erhält 117 g eines pulverförmigen Produkts mit einem Schmelzintervall von ca. 135-170 °C. Dieses Produkt entspricht der Formel (11) gemäss Beispiel 1.

Elementaranalyse :
ber. : C = 73,06 % H = 5,6 % Zn = 8,64 % O = 12,69 %
gef. : C = 71,8 % H = 5,7 % Zn = 8,54 % O = 12,35 %

Beispiel 4

465 g Salicylsäure, 735 g 1-Phenylethanol und 36 g p-Toluolsulfonsäure · 1$H_2O$ werden unter Rühren während 3 Stunden bei 120 °C kondensiert. Das gebildete Wasser wird laufend abdestilliert, wobei nach Ablauf von 3 Stunden noch während 30 Minuten bei reduziertem Druck destilliert wird. Danach wird auf 90 °C gekühlt und 1 770 ml einer wässrigen 2n-Natriumhydroxydlösung zugegeben. Die erhaltene Lösung wird einige Stunden bei Raumtemperatur stehen gelassen, worauf das an der Oberfläche abgeschiedene Oel abgetrennt wird. Hierauf lässt man die Lösung bei 10-15 °C und unter Rühren in eine Lösung von 290 g Zinkchlorid in 4 l Wasser zufliessen worauf der erhaltene weisse Niederschlag abfiltriert und abgepresst wird. Der feuchte Nutschkuchen wird nochmals in 5 l Wasser aufgeschlämmt, wieder abfiltriert, abgepresst und bei anfangs 50-70 °C im Vakuum getrocknet. Man erhält 965 g eines pulverförmigen Produktes mit einem Schmelzintervall von 105-140 °C. Dieses Produkt enthält 95 % des Zinksalicylats der

Formel (11) gemäss Beispiel 1 und 5 % eines Zinksalicylates der Formel

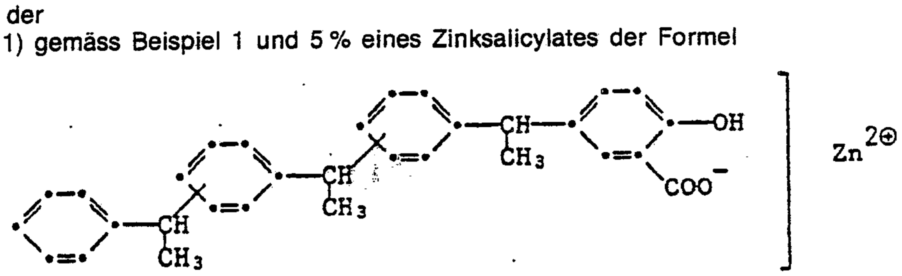

(13)

Elementaranalyse :
ber. : C = 73,06 % H = 5,0 % Zn = 8,64 % O = 12,69 %
gef. : C = 74,1 % H = 5,7 % Zn = 7,67 % O = 12,4 %

Beispiel 5

77,5 g Salicylsäure, 125 g 1-Phenylethanol und 6 g p-Toluolsulfonsäure · 1$H_2O$ werden unter Rühren

während 3 Stunden bei 120 °C kondensiert. Das gebildete Wasser wird laufend abdestilliert, wobei nach Ablauf von 3 Stunden noch während 15 Minuten bei reduziertem Druck destilliert wird. Danach wird auf 90 °C gekühlt und 295 ml einer wässrigen 2n Natriumhydroxydlösung zugegeben. Die erhaltene Lösung wird einige Stunden bei Raumtemperatur stehen gelassen, worauf das an der Oberfläche abgeschiedene Oel abgetrennt wird. Hierauf lässt man die Lösung bei 10-15 °C unter Rühren in eine Lösung von 36 g $Al_2(SO_4)_3 \cdot 18H_2O$ in 200 ml Wasser zufliessen, worauf der erhaltene weisse Niederschlag abfiltriert und abgepresst wird. Der feuchte Nutschkuchen wird nochmals in 400 ml Wasser aufgeschlämmt, wieder abfiltriert, abgepresst und bei 50-70 °C im Vakuum getrocknet. Man erhält 149,5 g eines pulverförmigen Produktes, welches das Aluminiumsalz der Formel

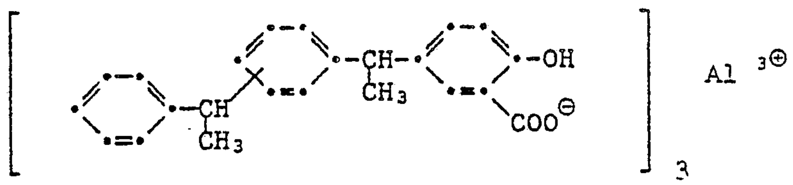

(14)

darstellt.

Elementaranalyse :

ber. : C = 77,88 % H = 5,97 % Al = 2,53 % O = 13,54 %

gef. : C = 77,6 % H = 6,15 % Al = 2,04 % O = 14,2 %

das Produkt enthält noch 0,9 % $H_2O$.

Beispiel 6

38,75 g Salicylsäure, 78,3 g 1-(2'-Chlorphenyl)-ethanol und 3 g p-Toluolsulfonsäure · $1H_2O$ werden unter Rühren während 3 Stunden bei 120 °C kondensiert. Das gebildete Wasser wird laufend abdestilliert, wobei nach Ablauf von 3 Stunden noch während 15 Minuten bei reduziertem Druck destilliert wird. Danach wird auf 90 °C gekühlt und 147,5 ml einer wässrigen 2n Natriumhydroxydlösung zugegeben. Die erhaltene Lösung wird einige Stunden bei Raumtemperatur stehen gelassen, worauf das als untere Phase ausgeschiedene Oel abgetrennt wird. Hierauf lässt man die Lösung bei 10-15 °C und unter starkem Rühren in eine Lösung von 24 g $ZnCl_2$ in 200 ml Wasser zufliessen, worauf der erhaltene Niederschlag abfiltriert und abgepresst wird. Der feuchte Nutschkuchen wird nochmals in 400 ml Wasser aufgeschlämmt, wieder abfiltriert, abgepresst und bei 20 °C im Vakuum getrocknet. Man erhält 70,4 g eines pulverförmigen Produkts der Formel

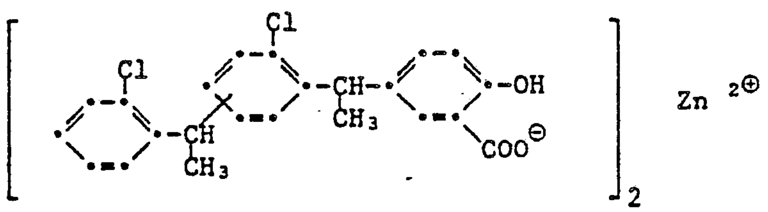

(15)

Dieses Produkt schmilzt bei 84-98 °C.

Elementaranalyse :

ber. : C = 61,20 % H = 4,23 % Cl = 15,36 % Zn = 7,31 %

gef. : C = 60,4 % H = 4,23 % Cl = 15,82 % Zn = 7,11 %

Beispiel 7

77,5 g Salicylsäure, 140,4 g 1-(p-Tolyl)-ethanol und 6 g p-Toluolsulfonsäure · $1H_2O$ werden unter Rühren während 3 Stunden bei 120 °C kondensiert. Das gebildete Wasser wird laufend abdestilliert, wobei nach Ablauf von 3 Stunden noch während 15 Minuten bei reduziertem Druck destilliert wird. Danach wird auf 90 °C gekühlt und 295 ml einer wässrigen 2n Natriumhydroxydlösung zugegeben. Die erhaltene Lösung wird einige Stunden bei Raumtemperatur stehen gelassen, worauf das an der Oberfläche abgeschiedene feste Nebenprodukt abfiltriert wird. Hierauf lässt man die Lösung bei 10-15 °C und unter starkem Rühren in eine Lösung von 50,3 g $ZnCl_2$ im 400 ml Wasser zufliessen, worauf der erhaltene Niederschlag abfiltriert und abgepresst wird. Der feuchte Nutschkuchen wird nochmals in 400 ml Wasser aufgeschlämmt, wieder abfiltriert, abgepresst und bei 50-70 °C im Vakuum getrocknet. Man erhält 149,4 g eines pulverförmigen Produktes der Formel

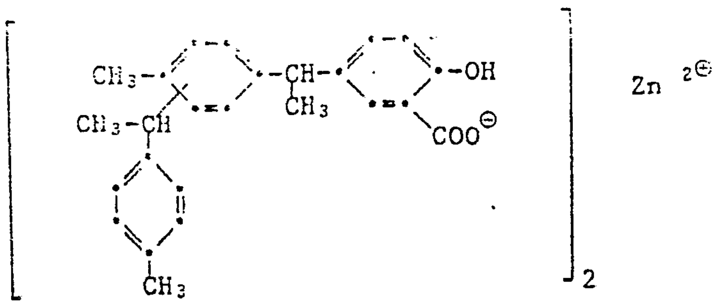

(16)

mit einem Schmelzintervall von 100-170 °C.

Anwendungsbeispiele

Beispiel 1 : (Druckempfindliches Aufzeichnungssystem)

Eine feingemahlene wässrige Dispersion (2-4 µm), die einen 38 %igen Feststoffgehalt aufweist, bestehend aus

1 Teil des gemäss Herstellungsbeispiel 1 hergestellten Zinksalzes der Formel (11)
13 Teilen China Clay
0,75 Teilen eines Kondensationsproduktes aus Naphthalinsulfonsäure und Formaldehyd und
1,5 Teilen eines Styrol/Butadiencopolymerisates (100 %)

wird auf ein Streichrohpapier mit einem Flächengewicht von 48 g/m² mit einem Rakel aufgetragen. Auftragsgewicht trocken : 6-7 g/m².

Die so hergestellte Nehmerschicht wird mit der Geberschicht eines handelsüblichen Durchschreibepapiers (z. B. Zanders) benachbart aufeinandergelegt. Die Geberschicht enthält in Mikrokapseln gelöst den Farbbildner, z. B. Kristallviolettlakton. Nach Durchschrift mit der Hand oder Schreibmaschine entsteht eine intensiv blaue Kopie.

Beispiel 2

Eine feingemahlene wässerige Dispersion (2-4 µm), die einem 38 %igen Feststoffgehalt aufweist bestehend aus

1 Teil des gemäss Herstellungsbeispiel 5 hergestellten Aluminiumsalzes der Formel (14)
6,5 Teilen China Clay
0,4 Teilen eines Kondensationsproduktes aus Naphthalinsulfonsäure und Formaldehyd
0,2 Teilen Polyvinylpyrrolidon
0,7 Teilen Styrol/Butadiencopolymerisat (100 %)

wird auf ein Streichrohpapier mit einem Flächengewicht von 48 g/m² mit einem Rakel aufgetragen. Auftragsgewicht trocken : 4-6 g/m².

Die so hergestellte Nehmerschicht wird mit der Geberschicht eines handelsüblichen Durchschreibepapiers (z. B. Zanders) benachbart aufeinandergelegt. Die Geberschicht enthält in Mikrokapseln gelöst den Farbbildner, z. B. Kristallviolettlakton. Nach Durchschrift mit der Hand oder Schreibmaschine entsteht eine intensiv blaue Kopie.

Gleich gute Ergebnisse werden erhalten, wenn anstelle des Aluminiumsalzes der Formel (14) die gemäss den Beispielen 3, 4, 6 und 7 hergestellte Zinksalze als Farbbildner verwendet werden.

Beispiel 3 (Thermographie)

Es werden zunächst zwei Dispersionen A und B hergestellt.

Zur Herstellung der Dispersion A werden

8 g des gemäss Herstellungsbeispiel 1 hergestellten Zinksalzes der Formel (11)
28 g einer 10 %igen wässrigen Lösung von Polyvinylalkohol 25/140 und
24 g Wasser

in einer Kugelmühle bis zu einer Korngrösse von 2-4 µm innerhalb 3 bis 6 Stunden gemahlen.

Zur Herstellung der Dispersion B werden

1 g Kristallviolettlakton,
3,5 g einer 10 %igen wässrigen Lösung von Polyvinylalkohol 25/140 und
4 g Wasser

in einer Kugelmühle bis zu einer Korngrösse von 2-4 µm gemahlen.

Anschliessend werden die beiden Dispersionen vermischt. Das farblose Gemisch wird auf ein Rohpapier mit einem Flächengewicht von 50 g/m² mit einem Rakel aufgetragen. Der Anteil des

aufgebrachten Materials beträgt 3 g/m² (Trockengewicht). Die Grundfarbe des so erhaltenen thermographischen Aufzeichnungspapiers ist farblos. Bei 125 °C entwickelt sich ein blauer Farbton, dessen volle Farbstärke bei 220 °C erreicht ist.

Intensive blaue Färbungen werden auch bei Verwendung der gemäss den Beispielen 2 bis 7 hergestellten Metallsalicyclate erhalten.

Beispiel 4 : Thermotransfer

a) Herstellung eines Bildübertragungsblattes A-1

Eine feingemahlene Dispersion (2-4 µm) der Zusammensetzung :

10 g Carnaubawachs
20 g Kristallviolettlakton
5 g Ethylcellulose
100 g Wasser

wird auf ein Rohpapier mit einem Flächengewicht von 50 g/m² mit einem Rakel aufgetragen. Auftragsgewicht : 7 g/m²

b) Herstellung eines Empfangsblattes B-1

Eine feingemahlene Dispersion (2-4 µm) der Zusammensetzung :

20 g des gemäss Beispiel 4 hergestellten Zinksalzes
10 g Siliciumdioxid-Pulver
30 g 10 %ige wässrige Polyvinylalkohol-Lösung
70 g Wasser

wird auf ein Rohpapier mit einem Flächengewicht von 50 g/m² mit einem Rakel aufgetragen. Auftragsgewicht : 4-5 g/m².

Das so hergestellte Bildübertragungsblatt A-1 wird mit dem Empfangsblatt B-1 Schicht gegen Schicht aufeinandergelegt. Der Rückseite des Bildübertragungsblattes wird über einen Thermodrucker während 0,3 Sekunden Wärme angeführt. Hierdurch entstehen auf dem Empfangsblatt B-1 ab 170 °C intensiv blaue Farbdrucke.

Intensive blaue Farbdrucke werden auch bei Verwendung der gemäss den Beispielen 1-3 und 5-7 hergestellten Metallsalicylate erhalten.

Beispiel 5 : (Druckempfindliches Aufzeichnungssystem)

Eine feingemahlene wässrige Dispersion (2-4 nm), die einen 38 %igen Feststoffgehalt bestehend aus

1 Teil eines Gemisches aus dem gemäss Herstellungsbeispiel 1 hergestellten Zinksalzes der Formel (11) und dem Zinksalz der 3,5-Bis-(α-methylbenzyl)-salicylsäure (Mischungsverhältnis 1 : 1)

7,4 Teilen China Clay

0,87 Teilen eines Kondensationsproduktes aus Naphthalinsulfonsäure und Formaldehyd

0,9 Teilen eines Styrol-Butadiencopolymerisates (100 %)

aufweist, wird auf ein Streichrohpapier mit einem Flächengewicht von 48 g/m² mit einem Rakel aufgetragen. Auftragsgewicht trocken : 6-7 g/m².

Die so hergestellte Nehmerschicht wird mit der Geberschicht eines handelsüblichen Durchschreibepapiers (z. B. Zanders) benachbart aufeinandergelegt. Die Geberschicht enthält in Mikrokapseln gelöst den Farbbilder, z. B. Kristallviolettlakton. Nach Durchschrift mit der Hand oder Schreibmaschine entsteht eine intensiv blaue Kopie.

**Patentansprüche**

1. Isomerengemische von Metallsalicylaten der Formel

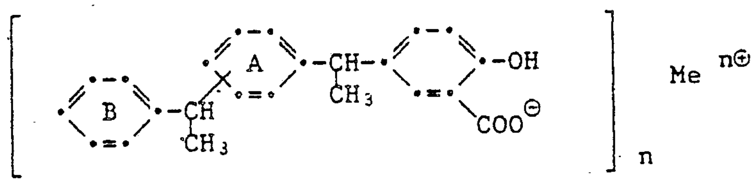

(1)

worin

Me ein n-wertiges Metallion und

n 2, 3 oder 4 bedeuten und die Ringe A und B unabhängig voneinander unsubstituiert oder durch Halogen, Niederalkyl, Niederalkoxy oder einen α-Methylbenzylrest substituiert sind.

2. Gemische gemäss Anspruch 1, dadurch gekennzeichnet, dass in Formel (1) Me das Aluminium-, Titan-, Vanadium-, Zinn oder vorzugsweise Zinkion ist.

3. Gemische gemäss einem der Ansprüche 1 und 2, dadurch gekennzeichnet, dass sie Aluminium- oder vorzugsweise Zinksalze einer substituierten Salicylsäureverbindung der Formel

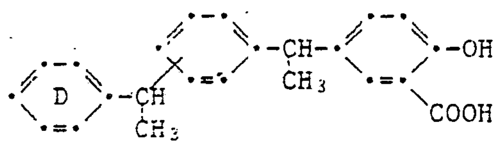

(2)

enthalten, in der der Ring D unsubstituiert oder durch α-Methylbenzyl substituiert ist.

4. Gemische gemäss Anspruch 3, dadurch gekennzeichnet, dass in Formel (2) der Ring D unsubstituiert ist.

5. Gemisch gemäss Anspruch 4, dadurch gekennzeichnet, dass es das Zinksalz der Salicylsäureverbindung der Formel

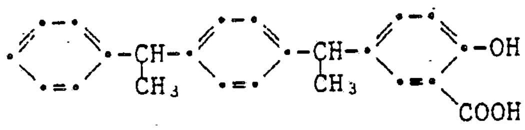

enthält.

6. Verfahren zur Herstellung der Metallsalicylate der im Anspruch 1 angegebenen Formel (1), dadurch gekennzeichnet, dass man 1 Mol des Salzes eines n-wertigen Metalls einer anorganischen Säure oder einer niederen aliphatischen Carbonsäure mit n Mol einer Salicylsäureverbindung der Formel

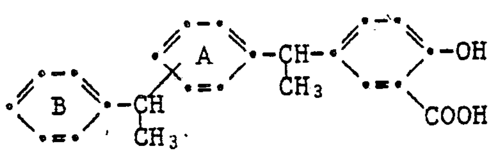

(3)

worin n, A und B die in Anspruch 1 angegebene Bedeutung haben, umsetzt.

7. Druckempfindliches oder wärmeempfindliches Aufzeichnungsmaterial, welches in seinem Farbreaktantensystem einen Farbbildner und einen Farbentwickler für den Farbbildner enthält, dadurch gekennzeichnet, dass der Farbentwickler mindestens ein Isomerengemisch von Metallsalicylaten der Formel

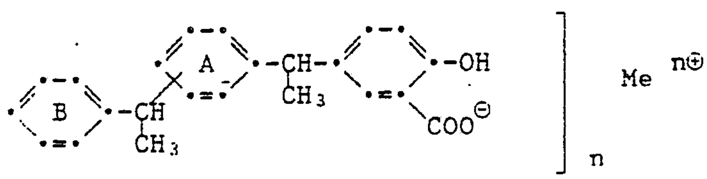

(1)

enthält, in der

Me ein n-wertiges Metallion und

n 2, 3 oder 4 bedeuten und die Ringe A und B unabhängig voneinander unsubstituiert oder durch Halogen, Niederalkyl, Niederalkoxy oder einen α-Methylbenzylrest substituiert sind.

8. Aufzeichnungsmaterial gemäss Anspruch 7, dadurch gekennzeichnet, dass der Farbentwickler ein Metallsalicylat der Formel (1) enthält, in der Me das Aluminium- oder vorzugsweise Zinkion ist.

9. Druckempfindliches Aufzeichnungsmaterial gemäss einem der Ansprüche 7 und 8, dadurch gekennzeichnet, dass es den Farbbildner gelöst in organischen Lösungsmitteln und eingekapselt in Mikrokapseln enthält.

10. Druckempfindliches Aufzeichnungsmaterial gemäss Anspruch 9, dadurch gekennzeichnet, dass der eingekapselte Farbbildner in Form einer Schicht auf der Rückseite eines Uebertragungsblattes und das Metallsalicylat der Formel (1) in Form einer Schicht auf der Vorderseite eines Empfangsblattes vorhanden sind.

11. Wärmeempfindliches Aufzeichnungsmaterial gemäss einem der Ansprüche 7 und 8, dadurch gekennzeichnet, dass es in mindestens einer Schicht mindestens einen Farbbildner, einen Farbentwickler und gegebenenfalls ein Bindemittel und/oder Wachs enthält, worin der Farbentwickler das in einem der Ansprüche 7 und 8 definierte Metallsalicylat enthält.

12. Isomerengemische von Salicylsäureverbindungen der Formel

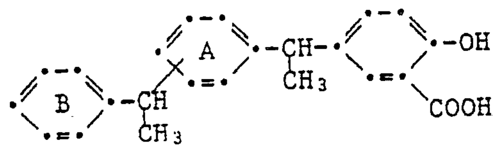

(3)

worin die Ringe A und B unabhängig voneinander unsubstituiert oder durch Halogen, Niederalkyl, Niederalkoxy oder einen α-Methylbenzylrest substituiert sind.

13. Verfahren zur Herstellung von Salicylsäureverbindungen der in Anspruch 12 angegebenen Formel (3), dadurch gekennzeichnet, dass man 1 Mol Salicylsäure mit mindestens 1 Mol eines 1-Phenylethanols der Formel

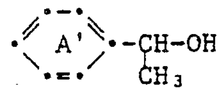

(4)

und mindestens 1 Mol eines 1-Phenylethanols der Formel

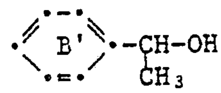

(5)

umsetzt, wobei die Ringe A' und B' unsubstituiert oder durch Halogen, Niederalkyl oder Niederalkoxy substituiert sind.

14. Verfahren gemäss Anspruch 13, dadurch gekennzeichnet, dass die Umsetzung in Gegenwart einer aromatischen Sulfonsäure, insbesondere p-Toluolsulfonsäure durchgeführt wird.

15. Verfahren gemäss Anspruch 14, dadurch gekennzeichnet, dass man eine in situ hergestellte aromatische Sulfonsäure, insbesondere Toluolsulfonsäure, verwendet.

**Claims**

1. An isomeric mixture of metal salicylates of the formula

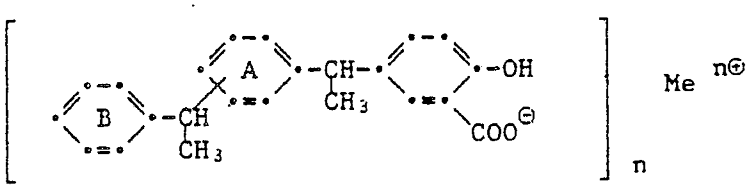

(1)

in which

Me is a metal ion of valency n,

n is 2, 3 or 4, and each of the rings A and B independently of the other is unsubstituted or substituted by halogen, lower alkyl, lower alkoxy or an α-methylbenzyl radical.

2. A mixture according to claim 1, wherein Me in formula (1) is the aluminium, titanium, vanadium, tin or preferably the zinc ion.

3. A mixture according to either of claims 1 and 2, which contains an aluminium salt or preferably a zinc salt of a substituted salicyclic acid compound of the formula

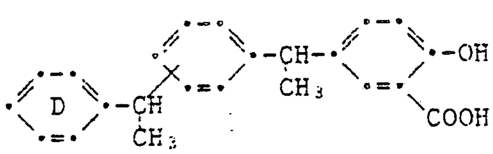

(2)

in which the ring D is unsubstituted or substituted by α-methylbenzyl.

4. A mixture according to claim 3, wherein the ring D in formula (2) is unsubstituted.

5. A mixture according to claim 4, which contains the zinc salt of the salicyclic acid compound of the formula

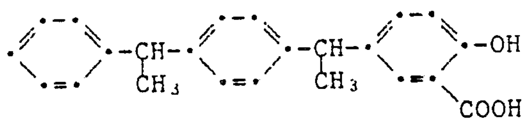

6. A process for the preparation of a metal salicylate of the formula (1) as indicated in claim 1, which comprises reacting 1 mole of a salt of an n-valent metal of an inorganic acid or of a lower aliphatic carboxylic acid with n moles of a salicylic acid compound of the formula

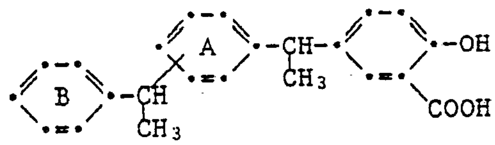 (3)

in which n, A and B are as defined in claim 1.

7. A pressure-sensitive or heat-sensitive recording material which contains in its colour reactive system a colour former and, as a developer for the colour former, at least one isomeric mixture of metal salicylates of the formula

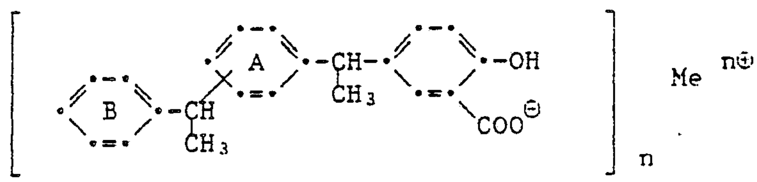 (1)

in which

Me is a metal ion of valency n,

n is 2, 3 or 4, and each of the rings A and B independently of the other is unsubstituted or substituted by halogen, lower alkyl, lower alkoxy or an α-methylbenzyl radical.

8. A recording material according to claim 7, wherein the developer is a metal salicylate of the formula (1) in which Me is the aluminium or preferably the zinc ion.

9. A pressure-sensitive recording material according to either of claims 7 and 8, which contains the colour former dissolved in an organic solvent and encapsulated in microcapsules.

10. A pressure-sensitive recording material according to claim 9, wherein the encapsulated colour former is present in the form of a layer on the back of a transfer sheet and the metal salicylate of the formula (1) is present in the form of a layer on the face of a receiver sheet.

11. A heat-sensitive recording material according to either of claims 7 and 8, which contains in at least one layer at least one colour former, one colour developer and, optionally, one binder and/or wax, where the colour developer contains a metal salicylate as defined in either of claims 7 and 8.

12. An isomeric mixture of salicylic acid compounds of the formula

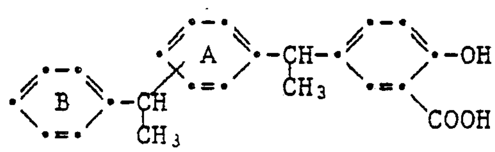 (3)

in which each of the rings A and B independently of the other is unsubstituted or substituted by halogen, lower alkyl, lower alkoxy or an α-methylbenzyl radical.

13. A process for the preparation of a salicylic acid compound of the formula (3) as indicated in claim 12, which comprises reacting 1 mole of salicylic acid with at least 1 mole of a 1-phenylethanol of the formula

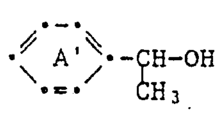 (4)

and at least 1 mole of a 1-phenylethanol of the formula

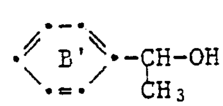 (5)

where the rings A' and B' are unsubstituted or substituted by halogen, lower alkyl or lower alkoxy.

14. A process according to claim 13, wherein the reaction is carried out in the presence of an aromatic sulfonic acid, in particular p-toluenesulfonic acid.

15. A process according to claim 14, which comprises using an aromatic sulfonic acid which is prepared in situ, in particular toluenesulfonic acid.

**Revendications**

1. Mélanges d'isomères de salicylates métalliques de formule

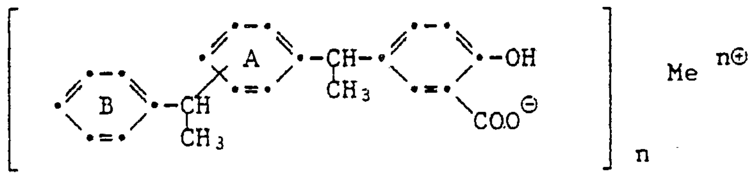

(1)

dans laquelle

Me représente un ion métallique de valence n, et

n est égal à 2, 3 ou 4 et les cycles A et B, indépedamment l'un de l'autre, sont non substitués ou substitués par un ou des halogènes, un ou des groupes alkyle inférieurs ou alcoxy inférieurs ou un groupe alpha-méthylbenzyle.

2. Mélanges selon la revendication 1, caractérisés en ce que, dans la formule (1), Me représente un ion aluminium, titane, vanadium, étain ou de préférence un ion zinc.

3. Mélanges selon l'une des revendications 1 et 2, caractérisés en ce qu'ils contiennent des sels d'aluminium ou, de préférence, du zinc, d'un dérivé substitué de l'acide salicylique répondant à la formule

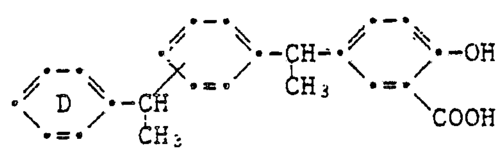

(2)

dans laquelle le cycle D est non substitué ou substitué par un groupe alpha-méthylbenzyle.

4. Mélanges selon la revendication 3, caractérisés en ce que, dans la formule (2), le cycle D est non substitué.

5. Mélange selon la revendication 4, caractérisé en ce qu'il contient le sel de zinc du dérivé de l'acide salicylique répondant à la formule

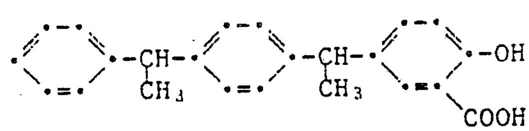

6. Procédé de préparation des salicylates métalliques de formule (1) donnée dans la revendication 1, caractérisé en ce que l'on fait réagir 1 mole du sel d'un métal de valence n d'un acide inorganique ou d'un acide carboxylique aliphatique inférieur avec n moles d'un dérivé de l'acide salicylique de formule

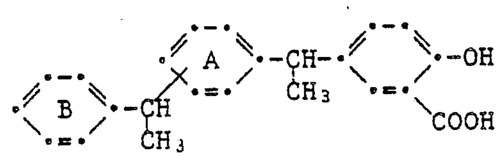

(3)

dans laquelle n, A et B ont les significations indiquées dans la revendication 1.

7. Matériau d'enregistrement sensible à la pression ou sensible à la chaleur, contenant dans son système de réactifs colorants un formateur de couleur et un révélateur de couleur pour le formateur de couleur, caractérisé en ce que le révélateur de couleur contient au moins un mélange d'isomères de salicylates métalliques de formule

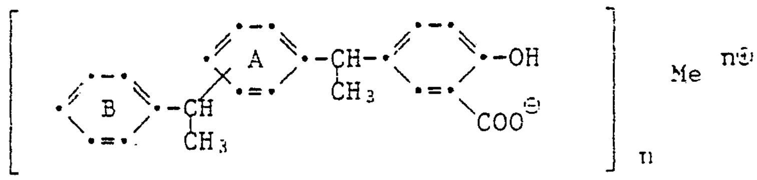

(1)

dans laquelle

Me représente un ion métallique de valence n, et

n est égal à 2, 3 ou 4, les cycles A et B, indépendamment l'un de l'autre, étant non substitués ou substitués par un ou des halogènes, un ou des groupes alkyle inférieurs ou alcoxy inférieurs ou un groupe alpha-méthylbenzyle.

8. Matériau d'enregistrement selon la revendication 7, caractérisé en ce que le révélateur de couleur contient un salicylate métallique de formule (1) dans laquelle Me représente l'ion aluminium ou, de préférence, l'ion zinc.

9. Matériau d'enregistrement sensible à la pression selon l'une des revendications 7 et 8, caractérisé en ce qu'il contient le formateur de couleur à l'état dissous dans des solvants organiques et encapsulé dans des microcapsules.

10. Matériau d'enregistrement sensible à la pression selon la revendication 9, caractérisé en ce que le formateur de couleur encapsulé est présent sous forme d'une couche sur le verso d'une feuille pour duplication et le salicylate métallique de formule (1) est présent sous forme d'une couche sur le recto d'une feuille réceptrice.

11. Matériau d'enregistrement sensible à la chaleur selon l'une des revendications 7 et 8, caractérisé en ce qu'il contient, dans une couche au moins, au moins un formateur de couleur, un révélateur de couleur et le cas échéant un liant et/ou une cire, et dans lequel le révélateur de couleur contient le salicylate métallique défini dans l'une des revendications 7 et 8.

12. Mélanges d'isomères de dérivés de l'acide salicylique de formule

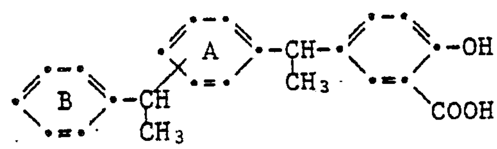

(3)

dans laquelle les cycles A est B, indépendamment l'un de l'autre, sont non substitués ou substitués par un ou des halogènes, un ou des groupes alkyle inférieurs ou alcoxy inférieurs ou un groupe alpha-méthylbenzyle.

13. Procédé de préparation des dérivés de l'acide salicylique de formule (3) donnée dans la revendication 12, caractérisé en ce que l'on fait réagir 1 mole d'acide salicylique avec au moins 1 mole d'un 1-phényléthanol de formule

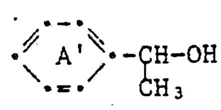

(4)

et au moins 1 mole d'un 1-phényléthanol de formule

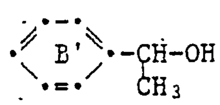

(5)

les cycles A' et B' étant non substitués ou substitués par un ou des halogènes, ou par un ou des groupes alkyles inférieurs ou alcoxy inférieurs.

14. Procédé selon la revendication 13, caractérisé en ce que la réaction est effectuée en présence d'un acide sulfonique aromatique, plus spécialement l'acide p-toluène-sulfonique.

15. Procédé selon la revendication 14, caractérisé en ce que l'on utilise un acide sulfonique aromatique préparé in situ, plus particulièrement l'acide toluène-sulfonique.